(19) Europäisches Patentamt
European Patent Office
Office européen des brevets

(11) Veröffentlichungsnummer: **0 477 828 B1**

# (12) EUROPÄISCHE PATENTSCHRIFT

(45) Veröffentlichungstag der Patentschrift: **01.02.95**

(21) Anmeldenummer: **91116165.1**

(22) Anmeldetag: **23.09.91**

(51) Int. Cl.[6]: **C12N 15/74**, C12N 9/02, C12P 17/00, C12N 1/20, //(C12N1/20,C12R1:19,1:38)

## (54) Hydroxylierung von Methylgruppen in aromatischen Heterocyclen mittels Mikroorganismen.

(30) Priorität: **24.09.90 CH 3066/90**

(43) Veröffentlichungstag der Anmeldung: **01.04.92 Patentblatt 92/14**

(45) Bekanntmachung des Hinweises auf die Patenterteilung: **01.02.95 Patentblatt 95/05**

(84) Benannte Vertragsstaaten: **AT BE CH DE DK FR GB IT LI NL**

(56) Entgegenhaltungen:
**EP-A- 0 277 674**
**WO-A-89/09823**

**CHEMICAL ABSTRACTS, vol. 95, no. 21, 23. November 1981, Columbus, Ohio, US; abstract no. 183729, BRODA, PAUL et al.: 'Degradative plasmids:TOL and beyond', Seite 336**

(73) Patentinhaber: **LONZA AG
(Geschäftsleitung: 4002 Basel)
CH-3945 Gampel/Wallis (CH)**

(72) Erfinder: **Zimmermann, Thomas, Dr.
Furkastrasse 9
Naters (Kanton Wallis) (CH)**
Erfinder: **Kiener, Andreas, Dr.
Meisenweg 5
Visp (Kanton Wallis) (CH)**
Erfinder: **Harayama, Shigeaki, Dr.
Rue du Grand Bay
Les Avanchets (Kanton Genf) (CH)**

(74) Vertreter: **Weinhold, Peter, Dr. et al
Patentanwälte Dipl.-Ing. G. Dannenberg
Dr. P. Weinhold
Dr. D. Gudel
Dipl.-Ing. S. Schubert
Dr. P. Barz
Siegfriedstrasse 8
D-80803 München (DE)**

EP 0 477 828 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, vol. 105, no. 11, 15. September 1986, Columbus, Ohio, US; abstract no. 94251u, WHITED, GREGORY M. et al.: 'Identification of cis-diols as intermediates in the oxidation of aromatic acids by a strain of Pseudomonas putida that contains a TOL plasmid', Seite 343

CHEMICAL ABSTRACTS, vol. 111, no. 17, 23. Oktober 1989, Columbus, Ohio, US; abstract no. 147542x, HARAYAMA, SHIGEAKI et al.: 'Characterization of five genes in the upper-pathway operon of TOL plasmid pWWO from Pseudomonas putida and identification of the gene products', Seite 147

## Beschreibung

Die Erfindung betrifft ein neues mikrobiologisches Verfahren zur Hydroxylierung von Methylgruppen in aromatischen 5- oder 6-Atom-Heterocyclen sowie für das Verfahren besonders geeignete neue Hybridplasmide und neue Produktionsstämme.

Diese hydroxymethylierten Heterocyclen sind beispielsweise wichtige Zwischenprodukte zur Herstellung von Pharmazeutika und Agrochemikalien.

Ein mikrobiologisches Verfahren zur terminalen Hydroxylierung von aliphatischen Seitenketten mittels gentechnologisch veränderten Mikroorganismen ist aus der EP-A 0 277 674 bekannt. Diese Reaktion wird durch die Alkan-Hydroxylase, kodiert durch die Gene alkBA aus dem OCT-Plasmid von Pseudomonas oleovorans katalysiert.
Diese Mikroorganismen wurden genetisch so verändert, dass sie nicht mehr befähigt sind, die entstandenen Hydroxylgruppen zur Säure weiter zu oxidieren. Die natürliche Expression und Regulation (alkR) dieser Gene wurde jedoch beibehalten.
Diese Mikroorganismen besitzen keine Aktivität für die Oxidation von Methylgruppen in Heterocyclen, sondern katalysieren nur die Hydroxylierung von Alkanen und alkylierten Verbindungen mit Alkylresten mit 6 bis 12 Kohlenstoffatomen.

Weiter ist aus Harayama et al., J.Bacteriol. 171, 1989, S.5048-5055 bekannt, dass Mikroorganismen der Spezies Pseudomonas putida mit dem Plasmid pWWO die Methylgruppe an Toluol in 3 Schritten zur Benzoesäure oxidieren können. Durch Einwirkung der Xylol-Monooxygenase (xylMA) entsteht dabei zuerst Benzylalkohol, welcher dann in zwei weiteren Schritten, katalysiert durch eine Alkohol- (xylB) und eine Aldehyddehydrogenase (xylC), zur Säure überführt wird.
In diesem Stamm liegen sowohl die xyl-Gene, die für die Enzyme des Xylol- Abbaus kodieren, als auch die Gene, welche für die Regulation der xyl-Gene verantwortlich sind auf dem Plasmid pWWO.
So sind daraus auch die Eigenschaften, die Identifikation, die Klonierung, die Selektion sowie die Restriktionskarten der für die Oxidation der Methylgruppe verantwortlichen Gene xylMABCN bekannt. Die Funktion des Gens xylN ist noch unbekannt. Es ist jedoch kein mikrobiologisches Verfahren bekannt, das Methylgruppen in aromatischen 5- oder 6-Atom-Heterocyclen hydroxylieren kann.
Chemisch sind solche spezifisch hydroxymethylierten Heterocyclen ausserdem nur schwer zugänglich.

Die Aufgabe der vorliegenden Erfindung bestand daher darin, ein mikrobiologisches Verfahren zur spezifischen Hydroxylierung von Methylgruppen in aromatischen 5- oder 6-Atom-Heterocyclen zu den entsprechenden reinen hydroxymethylierten Derivaten zu entwickeln, wobei die Produkte nicht weiter katabolisiert werden dürfen.

Diese Aufgabe wurde gemäss Patentanspruch 1 gelöst. Erfindungsgemäss wird das Verfahren mit Mikroorganismen durchgeführt, die

a) die Gene von einem Pseudomonas TOL-Plasmid enthalten, die eine aktive Xylol-Monooxygenase bilden

b) keine wirksame chromosomal- oder plasmid-codierte Alkohol-Dehydrogenase bilden

und damit befähigt sind, Methylgruppen von aromatischen 5- oder 6-Atom-Heterocyclen zum entsprechenden Hydroxymethylderivat zu hydroxylieren, wobei der Heterocyclus als Substrat für die Umsetzung dient und keinen Substituenten am benachbarten Kohlenstoffatom der zu hydroxylierenden Methylgruppe aufweist und das Hydroxymethylderivat nicht weiter katabolisiert wird.

Zweckmässig enthalten die Mikroorganismen die Gene zur Bildung einer Xylol-Monooxygenase von dem Pseudomonas TOL-Plasmid pWWO, die durch nachstehende Restriktionskarte charakterisiert und bereits in J.Bacteriol. 171 (1989), S.5048-5055 beschrieben sind:

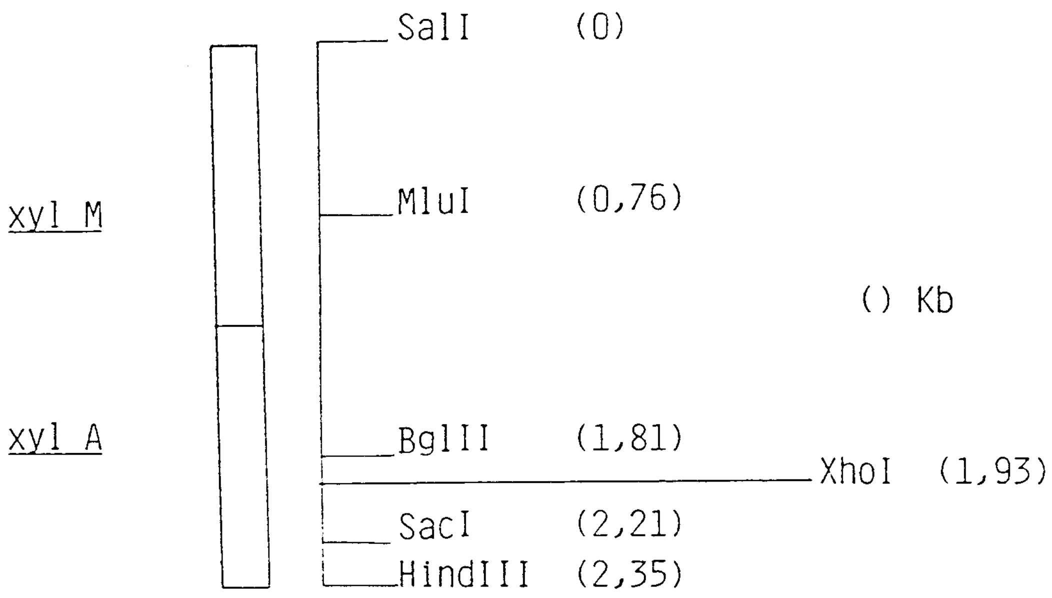


Quelle der Xylol-Monooxygenase-Gene

Als Quelle für die Xylol-Monooxygenase-Gene kann Pseudomonas putida mit dem TOL-Plasmid pWWO angewendet werden, der z.B. unter ATCC 33015 bei der American Type Culture Collection bezogen werden kann.

Die genetische Information, die für die Xylol-Monooxygenase codiert, kann dann dadurch erhalten werden, indem man a) die TOL-Plasmid-DNA aus diesem Mikroorganismus, der als Quelle für die DNA dient, isoliert, dann b) diese TOL-Plasmid-DNA zur Isolation des Gens für die Xylol-Monooxygenase verdaut und die spezifische Gen-Sequenz dann c) in einen Expressionsvektor einführt, wobei dadurch d) ein Hybridplasmid entsteht. Dieses Hybridplasmid kann dann in einen für das Verfahren geeigneten Mikroorganismus e) (Wirtsstamm) mittels Transformation f) eingebracht werden. Dieser transformierte Wirtsstamm bildet dann den Produktionsstamm g) (nach Selektion h)) für das erfindungsgemässe Fermentations-Verfahren i).

a) Isolation der TOL-Plasmid-DNA

Die TOL-Plasmid-DNA kann nach fachmännisch üblichen Methoden erhalten werden, wie beispielsweise nach der Methode von Hansen und Olsen (J.Bacteriol. 135, 1978, S.227-238) oder Humphreys et al. (Biochim. Biophys. Acta 383, 1975, S.457-483). Zweckmässig wird für die Isolation von grossen Mengen TOL-Plasmid-DNA die Methode von Humphreys et al. (Biochim. Biophys. Acta 383, 1975, S.457-483) angewendet, indem Pseudomonas putida (ATCC 33015) vollständig lysiert wird und anschliessend über Dichtegradientenzentrifugation das TOL-Plasmid isoliert wird.

b) Spaltung mit Restriktionsenzymen und Isolation der DNA durch Agarose-Gel-Elektrophorese

Zweckmässig wird nach Isolation der TOL-Plasmid-DNA die TOL-Plasmid-DNA mit den Restriktionsenzymen SalI und HindIII gespalten, wobei dann der DNA-Abschnitt der für die Xylol-Monooxygenase codiert, über Agarose-Gel-Elektrophorese, gemäss Current Protocols in Molecular Biology, John Wiley and Sons, New York, 1987, Abschnitt 2.6 "Isolation and Purification of Large DNA-Restriction Fragments from Agarose Gels", isoliert werden kann.
Dieser DNA-Abschnitt ist, wie zuvor schon beschrieben, durch nachstehende Restriktionskarte charakterisiert und enthält keine Gene, die für eine wirksame Alkoholdehydrogenase codieren:

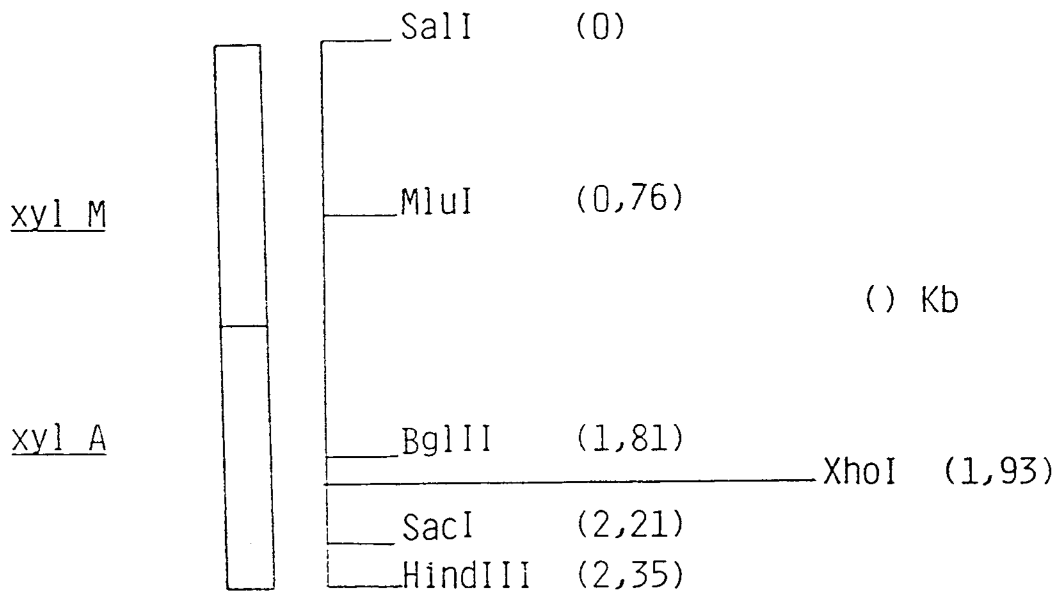


c) Ligation des DNA-Abschnitts in Expressionsvektoren

Der so erhaltene Genabschnitt kann mittels üblichen molekularbiologischen Techniken mit einer zuvor gleichermassen geschnittenen Expressionsvektor-DNA zu einem Hybridplasmid ligiert werden.
Expressions-Vektoren enthalten üblicherweise einen geeigneten, meist regulierbaren Promotor. Hinter diesem Promotor liegen günstigerweise in Transkriptionsrichtung eine oder mehrere singuläre Schnittstellen für Restriktionsenzyme. In diese Schnittstellen wird dann üblicherweise der gewünschte Genabschnitt inseriert, an dessen Expression man interessiert ist.
Zweckmässig werden als Expressionsvektoren alle, die in Tabelle 1 aufgeführt sind, angewendet. Für das erfindungsgemässe Verfahren werden zweckmässig Expressionsvektoren mit breitem Wirtsspektrum (broad host range) wie pME285, pKT240, pMMB67EH oder pMMB67EH* angewendet.
Zweckmässig werden diese Expressionsvektoren mit den Restriktionsenzymen SalI und HindIII geschnitten, und die entstandenen Restriktionsenden mit der isolierten TOL-Plasmid-DNA anschliessend mittels beispielsweise T4-DNA-Ligase ligiert. Gegebenenfalls können auch weitere Methoden zur Ligation angewendet werden, wie z.B. die, die in Current Protocols in Molecular Biology, John Wiley and Sons, New York, 1989, Abschnitt 3.16, Subcloning of DNA-fragments, beschrieben sind.

d) Hybridplasmide

Die zweckmässig so entstandenen Hybridplasmide pLO3, pLO4 und pLO5 sind ebenfalls Bestandteil der Erfindung, weisen ein breites Wirtsspektrum auf und können dementsprechend in Wirtsstämme mit hoher Substrat- und Edukttoleranz eingesetzt werden.
Zweckmässig sind diese Hybridplasmide vom natürlichen Regulationssystem entkoppelt.
Demgemäss wird Hybridplasmid pLO4 (bestehend aus Expressionsvektor pMMB67EH und dem TOL-Plasmid-Gen charakterisiert durch die zuvor beschriebene Restriktionskarte mit dem Promotor $P_{tac}$) kontrolliert durch das Repressorgen lacIq.
Die Expression der TOL-Plasmid-Gene lässt sich demnach mit Isopropylthiogalactosid (IPTG) induzieren.
Die Expression der TOL-Plasmid-Gene in Hybridplasmid pLO5 (bestehend aus Expressionsvektor pMMB67EH* und dem TOL-Plasmid-Gen charakterisiert durch die Restriktionskarte auf S. 4), mit Promotor $P_{tac}$, ist permanent (konstitutiv), aufgrund des fehlenden Repressorgens lacIq, induziert.
Zweckmässig wird hierfür durch Einführung einer Kanamycin-Resistenz das Repressorgen lacIq in pMMB67EH* mutiert. Ebenso ist es möglich ein Hybridplasmid mit einem engeren Wirtsspektrum anzuwenden. Zweckmässig wird als Hybridplasmid mit engerem Wirtsspektrum pGSH2836 mit Promotor lambda $P_L$ angewendet, wobei die Expression der TOL-Plasmid-Gene permanent (konstitutiv) induziert sind. Dient beispielsweise Escherichia coli (E.coli) K12* als Wirt für pGSH2836 muss durch Temperatur das dort chromosomal integrierte Repressorgen cI857 inaktiviert werden, um eine Expression des Promotors lambda

$P_L$ zu erreichen.
Das Hybridplasmid pGSH2836, ist hinterlegt in E.coli K12* unter der Nummer DSM 6154 bei der Deutschen Sammlung für Mikroorganismen und Zellkulturen GmbH, Mascheroderweg 1b, D-3300 Braunschweig. Die Hybridplasmide pLO4 und pLO5 sind wie in den folgenden Abschnitten beschrieben wird, in E.coli K12* (pLO4) oder in Pseudomonas putida (pLO5) hinterlegt.

e) Wirtsstämme

Aufgrund des breiten Wirtsspektrums können die so entstandenen Hybridplasmide (pLO3, pLO4, pLO5) in eine Vielzahl von Wirtsstämmen eingebracht werden.
Zweckmässig werden Wirtsstämme mit hoher Substrat- und Edukttoleranz eingesetzt wie z.B. die der Gattung Pseudomonas, Acinetobacter, Rhizobium, Agrobacterium oder Escherichia.

f) Transformation

Das Einbringen der Hybridplasmide in die zuvor beschriebenen Wirtsstämme kann nach üblichen Methoden erfolgen, vorzugsweise nach der Methode von Lederberg und Cohen (J.Bacteriol. 119, 1974, S.1072-1074).

g) Produktionsstämme

Als Produktionsstämme werden zweckmässig, alle die in Tabelle 2 aufgeführten angewendet. Die mit den Hybridplasmiden pLO3, pLO4 und pLO5 transformierten Mikroorganismen (Tabelle 2) sind neu und damit ebenfalls Bestandteil der Erfindung.
Vorzugsweise wird Mikroorganismus E.coli K12* transformiert mit Hybridplasmid pLO4, hinterlegt am 29.8.1990, unter der Nummer DSM 6153 oder Mikroorganismus Pseudomonas putida JD7 transformiert mit Hybridplasmid pLO5, hinterlegt am 29.8.1990, unter der Nummer DSM 6152 sowie deren Deszendenten und Mutanten, angewendet.
Die Hinterlegung erfolgte bei der Deutschen Sammlung von Mikroorganismen und Zellkulturen GmbH, Mascheroderweg 1b, D-3300 Braunschweig.
Es können auch solche Mikroorganismen als Produktionsstämme eingesetzt werden, die ein natürliches TOL-Plasmid enthalten und bei denen dann das Gen, welches für eine wirksame Alkoholdehydrogenase codiert, entfernt oder inaktiviert ist. Die Inaktivierung bzw. Entfernung kann durch klassische Mutation z.B. mit Acridin Orange über eine Transposon-Insertion oder durch die nachstehende Methode des "Gene-replacements" mit homologer Rekombination (A.Zimmermann et al, Molecular Microbiology 5, 1991, S.1483-1490) erfolgen.
In diesen Produktionsstämmen erfolgt dann die Expression der TOL-Plasmid-Gene beispielsweise durch Induktion mit Verbindungen wie Toluol, Xylol oder Cymol.

Die Entfernung des Alkohol-Dehydrogenase-Gens erfolgt zweckmässig derart, dass ein zuvor hergestelltes Hilfs-Hybridplasmid, in dem das Alkohol-Dehydrogenase-Gen bereits entfernt ist, durch homologe Rekombination in das natürliche TOL-Plasmid aufgenommen wird ("Gene-replacement" mit homologer Rekombination).
Durch diese Aufnahme wird dann zweckmässig die Alkohol-Dehydrogenase im natürlichen TOL-Plasmid entfernt.

h) Selektion der transformierten Mikroorganismen (Produktionsstämme)

Die Transformanten können üblicherweise auf Minimalmedium-Glucose-Agar mit entsprechender Inhibierungskonzentration geeigneter Antibiotika selektioniert werden. Die angewendeten Antibiotika-Resistenzmarken sind in Tabelle 1 aufgeführt.

i) Fermentationsverfahren

Erfindungsgemäss werden die nach den zuvor beschriebenen Verfahren erhaltenen Produktionsstämme sowie deren Deszendenten und Mutanten für das erfindungsgemässe Verfahren zur Hydroxylierung von Methylgruppen in aromatischen 5- oder 6-Atom-Heterocyclen eingesetzt.
Als Substrate für die Umsetzung können methylierte aromatische 5- oder 6-Atom-Heterocyclen verwendet werden, die ein oder mehrere Heteroatome aus der Reihe Sauerstoff, Stickstoff, Schwefel enthalten.

Geeignete 5-Atom-Heterocyclen sind beispielsweise methylierte Thiophen-, Furan-, Pyrrol-, Thiazol-, Pyrazol- und Imidazol-derivate, die keinen Substituenten am benachbarten Kohlenstoffatom der zu hydroxylierenden Methylgruppe aufweisen. Vorzugsweise werden als 5-Atom-Heterocyclen, 3,5-Dimethylpyrazol, 4-Methylthiazol und 2,5-Dimethylthiophen, angewendet.
Geeignete 6-Atom-Heterocyclen sind beispielsweise methylierte Pyridin-, Pyrimidin-, Pyrazin- und Pyridazin-derivate, die keinen Substituenten am benachbarten Kohlenstoffatom der zu hydroxylierenden Methylgruppe aufweisen. Vorzugsweise werden als 6-Atom-Heterocyclen, 2-Chlor-5-methylpyridin, 2,5-Dimethylpyrazin und 2,6-Dimethylpyrimidin, angewendet.
Vor der Substratzugabe werden die Zellen bis zu einer optischen Dichte bei 650 nm ($OD_{650}$) von 1 bis 200 im Kulturmedium angezogen, vorzugsweise bis zu einer optischen Dichte von 5 bis 100.
Die Umsetzung kann entweder unter einmaliger oder kontinuierlicher Substratzugabe erfolgen, so dass die Substratkonzentration im Kulturmedium 20% (w/v) bzw. (v/v) für flüssige Substrate nicht übersteigt.
Vorzugsweise erfolgt die Substratzugabe so, dass die Substratkonzentration im Kulturmedium 5% (w/v) bzw. (v/v) nicht übersteigt.
Die Umsetzung wird üblicherweise mit ruhenden Zellen in einem pH-Bereich von 4 bis 11, vorzugsweise von 6 bis 10, durchgeführt.
Die Umsetzung wird üblicherweise bei einer Temperatur von 15 bis 50 °C, vorzugsweise bei einer Temperatur von 25 bis 45 °C, durchgeführt.
Nach der Umsetzung können die entsprechenden Hydroxymethylderivat auf bekannte Art und Weise isoliert werden.

## Beispiel 1

## Klonierung der Gene xylMA

### 1.1. Plasmidpräparation

(Humphreys et al., Biochim. Biophys. Acta 383 (1975), S.457-483).

Die Zellen von 1 l einer ausgewachsenen Bakterienkultur, Pseudomonas putida pWWO (ATCC 33015), wurden abzentrifugiert. Nach Resuspension der Zellen in 10 ml 25% Saccharose in 0,05 M Tris-Puffer, pH 8,0 wurden 1,5 ml Lysozymlösung, (20 mg/ml in 0,25 M Tris-Puffer, pH 8,0) hinzugegeben. Anschliessend Inkubation für 5 min. auf Eis; Zugabe von 10 ml 0,25 M $Na_2EDTA$ (pH 8) und weitere Inkubation für 5 min. auf Eis.
Dann Zugabe von 15 ml Brij® Polyoxyethylenlaurylether/DCL-Lösung (1% Brij® 58, 0,4% Natriumdeoxycholat in 0,01 M Tris-Puffer, 0,001 M $Na_2EDTA$, pH 8,0). Gutes, gleichmässiges Durchmischen, Inkubation auf Eis für 30 min. bis zur vollständigen Zellyse.
Nach Zentrifugation für 45 min. bei 4 °C bei 16 000 UpM, wurde der Überstand in autoklavierten Messzylinder dekantiert. Zugabe von 3% (w/v) NaCl und 10% PEG (Polyethylenglykol). Durch vorsichtiges Wenden des mit Parafilm verschlossenen Zylinders wurde eine Lösung erzielt. Inkubation für 2 h bei 4 °C. Zentrifugation für 2 min bei 5 000 UpM. Anschliessend Dekantieren des Ueberstandes und Lösen des Niederschlags in 5 ml TES- Puffer (0,05 M Tris, 0,005 M $Na_2$ EDTA, 0,05 M NaCl, pH 8,0); Überführung in, autoklaviertes 15 ml Corex-Röhrchen mit 8,0 g Calciumchlorid. Nach Zugabe von 0,6 ml Ethidiumbromidlösung (10 mg/ml) wurde für 30 min auf Eis inkubiert.
Zentrifugation für 30 min bei 4 °C bei 12 000 UpM. Dann wurde vorsichtig dekantiert zur Entfernung des ausgefallenen PEG aus der Lösung. Ultrazentrifugation der Lösung in geschlossenen Röhrchen mit 50TI-Rotor bei 40 000 UpM für 30 h bei 18 °C. Danach Isolierung der Plasmidbande aus den $CsCl_2$-Gradienten mit einer Kanüle vor dem UV-Transilluminator.
Entfernung von Ethidiumbromid aus dem Plasmidpräparat durch Ausschütteln mit n-Butanol. Isopropanolfällung der Plasmid-DNA und Trocknen des Niederschlags im Speed Vac® Concentrator. Resuspension des Plasmidpräparats in 0,01 M Tris-Puffer, 0,001 M $Na_2$ EDTA, pH 8,0).

### 1.2 Isolation von DNA-Fragmente xylMA aus Agarose-Gelen

Die mit SalI und HindIII (jeweils 4 Units pro $\mu$g Plasmid-DNA) geschnittene Plasmid-DNA wurde einer präparativen Agarose-Gelelektrophorese unterworfen, (0,6% (w/v) Agarose in TBE-Puffer (0,09 M Tris-Borat, 2,5 mM $Na_2$ EDTA, pH 8,3, Ethidiumbromid (100 $\mu$g/100 ml)).
In kleine Streifen geschnittene DEAE-Cellulose-Membran wurde in $H_2O$ angerichtet und in Schlitze im

Agarosegel unmittelbar vor der gewünschten DNA-Fragmentbande gesteckt. DNA wurde im Spannungsfeld auf die Membran auflaufen gelassen. Gegebenenfalls höhere DNA-Banden wurden mit weiterer Membran zurückgehalten. Die aufgelaufene DNA wurde von der Membran mit 500 µl Elutionspuffer (20 mM Tris, pH 7,5, 1 mM $Na_2$ EDTA, 1,5 M NaCl) während 1 h bei 65 °C abgewaschen.
Membran wurde entfernt und abgespült. Ethidiumbromid wurde mit $H_2O$-gesättigtem n-Butanol aus der DNA-Lösung extrahiert, DNA mit Isopropanol präzipitiert.
Trocknen des Niederschlags im Speed Vac® Concentrator. Resuspension des Fragmentpräparats in 0,01 M Tris-Puffer, 0,001 M $Na_2$ EDTA, pH 8,0.

1.3 Ligation von DNA-Fragmenten xylMA mit den Expressionsvektoren

(Current Protocols in Molecular Biology, John Wiley and Sons, New York (1989), Abschnitt 3.16, Subcloning of DNA-fragments)

a) Präparation von Hybridplasmid pLO4 Vorbereiten der Expressionsvektor-DNA

Vor der Ligation wurde die pMMB67EH-Vektor-DNA (2µg) mit je 10 Units SalI und HindIII in dem entsprechenden Ligationspuffer (20 mM Tris-Puffer, 10 mM DTT (Dithioerythritol), 10 mM $MgCl_2$, 0,6 mM ATP, pH 7,2) geschnitten.
Diese geschnittene DNA wurde anschliessend mit 4,8 Units alkalischer Phosphatase dephosphoriliert. Die DNA wurde wiederholt mit Isopropanol ausgefällt und gewaschen.

Ligation der xylMA-DNA mit der Expressionsvektor-DNA

Zur Ligation wurden die jeweiligen DNA-Proben (in verschiedenen Mengenverhältnissen bei Ueberschuss der Insert-DNA) zusammengegeben, einer Isopropanolfällung unterzogen, und die getrockneten Niederschläge wurden in 40 bis 100 µl Ligationspuffer aufgenommen (20 mM Tris-Puffer, 10 mM DTT, 10 mM $MgCl_2$, 0,6 mM ATP, pH 7,2). Die Ligation erfolgte nach Zugabe von 0,2 Units T4-DNA-Ligase pro µg DNA über Nacht unter Inkubation bei 12 bis 16 °C.
Anschliessend wurde das Ligationsgemisch direkt zur Transformation eingesetzt.

b) Präparation von Hybridplasmid pLO5

Analog zu Beispiel 1.3 a) wurde der Expressionsvektor pMMB67EH* vorbereitet und entsprechend zu Beispiel 1.3 a) wurde dann die xylMA-Gene in diesen Vektor ligiert.

1.4 Transformation kompetenter Zellen mit Hybridplasmid-DNA (pLO4)

a) Anreicherung der Hybridplasmid-DNA (pLO4)

Die Zellen einer 25 ml-Kultur von E.coli S17-1 als Hilfsstamm wurden bei einer $OD_{546}$ = 2,0 geerntet und nach der Methode von Lederberg und Cohen (J.Bacteriol. 119 (1974), 1072-1074) kompetent gemacht.
Nach Waschen dieser Zellen in 10 ml 0,1 M $MgCl_2$ wurden die Zellen für 30 min in 10 ml 0,1 $CaCl_2$ auf Eis inkubiert.
Zentrifugation und Resuspension dieser Zellen in 1 ml 0,1 M $CaCl_2$. Je 0,2 ml der Zellsuspension wurden mit 0,5 µg ligierter Hybrid-Plasmid-DNA zur Transformation vermischt. Inkubation für mehr als 30 min auf Eis, 2-minütiger Hitzeschock bei 42 °C.
Anschliessend wurden die jeweiligen Zellsuspensionen auf 5 ml mit vorgewärmten Nutrient Yeast Broth (Oxoid, Wesel, BRD) aufgefüllt, für 1 h ohne Schütteln und eine weitere Stunde mit Schütteln zur Expression der Gene bei optimaler Wachstumstemperatur der Rezipientenzellen (E.coli S17-1) inkubiert. Aliquots der transformierten Kulturen wurden auf entsprechende Selektivmedien (Nutrient Agar, 100 µg Ampicillin pro ml) plaziert.

b) Transformation von pLO4 in den Produktionsstamm

Aus dem E.coli S17-1-Stamm mit pLO4 wurde das Hybridplasmid pLO4 entsprechend zu Beispiel 1.1 und 1.2 isoliert. Anschliessend wurde E.coli K12* entsprechend der Methode in Beispiel 1.4 a) mit dem Hybridplasmid pLO4 transformiert. Die Selektion erfolgte entsprechend mit dem Selektivmedium (Nutrient

Agar, 100 µg Ampicillin pro ml)

1.5 Transformation kompetenter Zellen mit Hybridplasmid pLO5

Entsprechend zu Beispiel 1.4 wurde das Hybridplasmid pLO5 in Pseudomonas putida JD7 transformiert.
Die Selektion erfolgte mit Selektivmedium (Nutrient Agar, 50 µg Kanamycin pro ml)

Beispiele 2 bis 8

Entsprechend zu Beispiel 1.3 wurde Hybridplasmid pLO3 hergestellt.
Entsprechend zu den Beispielen 1.4 und 1.5 wurden die Hybridplasmide pGSH2836, pLO3, pLO4 und pLO5 in die Wirtsstämme E.coli K12*, Pseudomonas aeruginosa PAO25, Pseudomonas putida JD7 und Pseudomonas putida, transformiert.
Die Umsetzungsraten dieser Produktionsstämme sind in Tabelle 2 zusammengefasst.

Beispiel 9

Konstruktion der xylB-Mutante

9.1. Konstruktion von Plasmid pLO10

Die Gene xylMABCN wurden aus dem Plasmid pGSH2816 (Haramaya et al., J.Bacteriol. 171, 1989) mittels EcoRI und HpaI-Restriktion isoliert und anschliessend in den gleichermassen geschnittenen Vektor pBR322 (Bolivar et al., Gene 2, 1977, S.95 ff) ligiert.

9.1.1 Spaltung mit Restriktionsenzymen und Isolation der DNA durch Agarose-Gelelektrophorese

Die mit EcoRI und HpaI (jeweils 5 Units pro µg DNA) geschnittene pGSH2816-DNA wurde mittels präparativer Agarose-Gelelektrophorese aufgetrennt (0,7% Agarose in 0,09 M Tris-Borat, 2,5 mM Na-EDTA, pH 8,3) und die DNA-Fragmente der gesuchten Grösse wurden isoliert (entsprechend zu Beispiel 1.2).

9.1.2 Ligation des DNA-Fragmentes mit xylMABCN in pBR322

(Current Protocols in Molecular Biology, John Wiley and Sons, New York 1988: Abschnitt 3.16, Subcloning of DNA-fragments)

a. Vorbereiten der Vektor-DNA

Vor der Ligation wurde die pBR322-DNA (2 µg) mit je 10 Units EcoRI und ScaI in dem Restriktionspuffer (50 mM Tris, 10 mM $MgCl_2$, 100 mM präparativen Agarose-Gelelektrophorese getrennt. Die gewünschte 3850 bp grosse Bande wurde, wie in Beispiel 9.1.1 beschrieben, isoliert.

b. Ligation

Zur Ligation wurden die jeweiligen DNA-Proben (in verschiedenen Mengenverhältnissen bei Überschuss der Insert-DNA) zusammengegeben, mit Ligationspuffer versetzt (20 mM Tris, 10 M DTT, 10 mM $MgCl_2$, 0,6 mM ATP, pH 7,2) und nach Hinzufügen von 1 Unit T4-DNA-Ligase über Nacht bei 12 bis 16°C inkubiert. Anschliessend wurde das Ligationsgemisch direkt zur Transformation eingesetzt.

c. Transformation von E.coli C600

(entsprechend zu Beispiel 1.4)

Die Selektion erfolgte auf Nutrient Agar mit Tetracyclin (25 µg/µl). Nach Restriktionskontrolle wurden grössere Mengen von pLO10-DNA mittels CsCl-Gradient aufgereinigt.

9.2 Konstruktion von Plasmid pLO11

9.2.1 Spaltung von pLO10-DNA mit Restriktionsenzymen

5μg pLO10-DNA wurden mit 22 Units HindIII im Restriktionspuffer geschnitten (10 mM Tris, 10 mM $MgCl_2$, 50 mM NaCl, 1 mM DTT, pH 7,5) und einer präparativen Agarose-Gelelektrophorese unterworfen. Zwei Fragmente mit Grössen von 3,8 kb bzw. 2,9 kb wurden, wie in Beispiel 9.1.1 beschrieben, isoliert und in 45 bzw. 30 μl Wasser aufgenommen. Sie enthielten den Vektor pBR322 und den Bereich der xyl-Gene ausser xylB.

9.2.2 Ligation

Beide der in Beispiel 9.2.1 isolierten Fragmente wurden, wie in Beispiel 9.1.2b beschrieben, zur Ligation eingesetzt. Dazu wurden 45 μl Fragment 3,8 kb, 30 μl Fragment 2,8 kb, 10 μl Ligationspuffer, 10 μl 10 mM ATP und 1 μl T4-DNA-Ligase gemischt und über Nacht bei 12 bis 16 °C inkubiert. Anschliessend wurde die DNA mit Ethanol gefällt und in 10 μl Wasser aufgenommen.

9.2.3 Transformation von E.coli HB101

Die nach Beispiel 9.2.2 erhaltene DNA wurde direkt zur Transformation von E.coli HB101 verwendet (entsprechend zu Beispiel 9.1.2c).
Transformierte Zellen wurden auf Nutrient Agar mit Tetracyclin (25 μg/μl) selektioniert.

9.3 Überführung von pLO11 in E.coli HB101 enthaltend pRK2013

E.coli HB101 enthaltend pRK2013 wurde als Wirt für pLO11 ausgewählt. Mittels der auf pRK2013 codierten Funktionen ist eine Mobilisierung in andere gram-negative Bakterien wie z.B. Pseudomonas putida JD7 mit pWW0 möglich.
Isolierte pLO11-DNA wurde wie in Beispiel 9.1.2a beschrieben in E.coli HB101 enthaltend pRK2013 transformiert. Die Selektion erfolgte auf Nutrient Agar mit Tetracyclin (25 μg/μl) und Kanamycin (25 μg/μl).

9.4 Konjugation von E.coli HB101 enthaltend pRK2013 pLO11 mit Pseudomonas putida JD7 enthaltend pWW0

Von Übernacht-Kulturen der beiden Konjugationspartner wurden 2 ml abzentrifugiert, mehrfach in 0,9% NaCl (Saline) gewaschen, in 100 μl Saline aufgenommen und auf Nutrient Agar Platten gemischt. Die Platten wurden zur Konjugation für 6 h bei 30 °C inkubiert. Der entstandene Bakterienrasen wurde in 1 ml Saline resuspendiert und in geeigneten Verdünnungen auf Nutrient Agar mit Tetracyclin (50 μg/μl) plattiert. Unter den erhaltenen Transkonjuganten sollten einige aufgrund homologer Rekombination pLO11 in das TOL-Plasmid aufgenommen haben.

9.5 Markeraustausch zwischen pLO11 und pWW0

Um mittels homologer Rekombination xylB auf dem TOS-Plasmid pWW0 in Pseudomonas putida JD7 zu entfernen, wurden ca. über 100 Generationen der zuvor erhaltenen E.coli-Transkonjuganten ohne Selektionsdruck durch Tetracyclin angezogen (kultiviert). Dabei war die Exclusion (Entfernung) von Vektor pBR322 und intaktem xylB-Gen erwünscht.
Um die Anzahl Tetracyclin-sensitiver xylB-Mutanten zu erhöhen, wurde dann eine Selektion gegen den integrierten Vektor pBR322 durchgeführt:
Zellen wurden in 25 ml Komplexmedium Nutrient Yeast Broth (Oxoid, Wesel, BRD) Tetracyclin (50 μg/μl) aufgenommen und bis zu einer $OD_{650}$ von ca. 3,0 bei 30 °C inkubiert. Danach wurden 500 μg/ml Cycloserin C und 100 μg/ml Piperacillin zugegeben. Nach mehrstündiger Inkubation bei 30 °C trat eine fast vollständige Lyse der Zellen ein. Überlebende Zellen wurden abzentrifugiert, mehrfach in Saline gewaschen und in geeignete Verdünnungen auf Nutrient Agar plattiert. Bis zu 85% der erhaltenen Kolonien waren Tetracyclin-sensitiv.

9.6 Test der Kolonie auf Anwesenheit einer xylB-Deletion

9.6.1 Nachweis der Deletion mit Southern-Blot-Hybridisierung

pWW0'-DNA der erhaltenen Klone wurde entsprechend der Methode von Kado und Liu (J.Bacteriol. 145, S.1365-1373 (1981)) isoliert. Dazu wurde 1 ml Übernacht-Kultur abzentrifugiert und in 40 mM Tris-Acetat-Puffer, 2 mM EDTA, pH 7,9 resuspendiert. Die Zellen wurden durch Zugabe von 200 µl 3% SDS, pH 12,6 lysiert, 1 h bei 65 °C inkubiert und anschliessend mehrfach mit Phenol-Chloroform (1:1) extrahiert.
Die wässrige DNA-Lösung wurde durch wiederholtes Waschen mit Diethylether von Phenol befreit und mit 1/10 Volumen 3 M Natrium-Acetat, pH 4,8 versetzt.
Danach wurde die DNA mit Ethanol präzipitiert und nach Trocknen in 100 µl Wasser aufgenommen.
Ca. 40 µl dieser DNA-Probe wurden mit 100 Units EcoRI und 5 Units HpaI im Verdauungspuffer (50 mM Tris, 10 mM $MgCl_2$, 100 mM NaCl, 1 mM DTT, pH 7,5) geschnitten und einer Agarose-Gelelektrophorese unterworfen. Die auf Nitrocellulose-Membranen transferierte DNA wurde gegen 500 ng einer $^{32}$P-ATP markierten pLO11 Sonde hybridisiert. Die 1,4 kb xylB-Deletion war nach Autoradiographie direkt erkennbar.

Beispiel 10

Herstellung der hydroxymethylierten Heterocyclen

E.coli K12* mit pLO4 (DSM-Nr. 6153) wurden über Nacht bei 30 °C in Nutrient Yeast Broth (Oxoid, Wesel, BRD) unter Zusatz von dem in Tabelle 1 aufgeführten entsprechenden Antibiotikum entsprechend der Methode in Beispiel 1.4 zur Stabilisierung der Plasmide angezogen. Danach wurde ein Aliquot in frisches Medium übertragen und für weitere 2 h bei 30 °C inkubiert, bevor die Xylol-Monooxygenase-Gene entsprechend dem Expressionssystem (Tabelle 1) induziert wurden. Dies geschah durch Zugabe von 1 mM IPTG zur Induktion der Expression durch den tac-Promotor. Die Induktionsphase betrug jeweils zwischen 2 und 4 h. Die Bakteriensuspension wurde zentrifugiert, und das Zellpellet dann in frisches Medium ohne Zusatz von Antibiotika resuspendiert, so dass sich eine $OD_{650}$ von 10 einstellte. Diese Suspension wurde danach mit 0,1% (v/v für flüssige Substrate, w/v für feste Substrate) der zu oxidierenden Heterocyclen versetzt und bei 30 °C weiterinkubiert. Nach bestimmten Zeitabständen wurde die Bakteriensuspension auf Produktbildung hin untersucht.

Beispiel 11

Entsprechend zu Beispiel 10 wurde Pseudomonas putida JD7 mit pLO5 angezüchtet.
Aufgrund des fehlenden Repressorgens lacIq konnte auf eine Induktion mit IPTG verzichtet werden.
Der Stamm wurde entsprechend zu Beispiel 10 zur Umsetzung der Heterocyclen eingesetzt.

Beispiel 12

Entsprechend zu Beispiel 10 wurde E.coli K12* mit pGSH2836 (DSM-Nr. 6154) für die Umsetzung eingesetzt.
Die Induktion erfolgte durch Inaktivierung des Repressorgens cI857 durch Temperatureinwirkung für 2 h bei 42 °C.

Beispiele 13 und 14

Entsprechend zu Beispiel 10 wurden die in den Beispielen 2 bis 8 hergestellten Produktionsstämme für die Umsetzung eingesetzt.

Beispiele 15 bis 20

Die Ergebnisse der Umsatzraten der verschiedenen Heterocyclen mit dem Produktionsstamm von Beispiel 12, sind in Tabelle 3 zusammengefasst.

T a b e l l e   1

| Expressions-vektoren (ohne xylMA) | beschrieben in | Hybridplasmide mit xylMA | charakterisiert durch | Grösse in kb |
|---|---|---|---|---|
| pLV85 | J.Bacteriol.169 (1987) S.4457-4462 | pGSH2836 | Ampicillin-resistent Promotor lambda PL | 5,25 |
| pME285 | Gene 36 (1985) S.27-36 | pLO2 | Kanamycin-sensitiv Quecksilbersalzresistent$^+$ mob$^-$ | 12,95 |
| pKT240 | Gene 26 (1983) S.273-282 | pLO3 | Kanamycin-sensitiv Ampicillin-resistent$^+$ mob$^-$ | 15,25 |
| pMMB67EH | Gene 48 (1986) S.119-131 | pLO4 | Ampicillin-resistent Promoter $P_{tac}$ lacIq$^+$ | 11,15 |
| pMMB67EH* | - | pLO5 | Ampicillin-resistent Promotor $P_{tac}$ lacIq$^-$ Kanamycin-resistent | 13,5 |

<u>T a b e l l e   2</u>: Produktionsstämme zur Hydroxylierung von Methylgruppen

| Beispiele | Produktions-Stämme | enthaltend Hybridplasmid oder mutiertes Plasmid (pWWO') | Hinterlegungsnummer DSM | Ausbeute in % bei d.Umsetzung v.2-Chlor-5-methyl-pyrimidin als Substrat in einer Konzentration von 0,1% (v/v) |
|---|---|---|---|---|
| 2 | <u>E.coli</u> K12* | pGSH2836 | 6154 | 80 |
| 3 | <u>E.coli</u> K12* | pLO4 | 6153 | 80 |
| 4 | <u>E.coli</u> K12* | pLO3 | - | 80 |
| 5 | <u>E.coli</u> K12* | pLO5 | - | 80 |
| 6 | <u>Pseudomonas aeruginosa</u> PAO25 | pLO5 | - | 10 |
| 7 | <u>Pseusomonas putida</u> JD7 | pLO5 | 6152 | 5 |
| 8 | <u>Pseudomonas putida</u> | pLO5 | - | 5 |

Tabelle 3: Mikrobiologische Oxidation methylierter aromatischer Heterocyclen mit Mikroorganismenstamm: *E.coli* K12* enthaltend den Expressionsvektor pGSH2836

| Beispiel | Substrat | Konzentration des Substr. im Kulturmedium | Reaktionszeit in h | Endprodukt | Ausbeute in % |
|---|---|---|---|---|---|
| 15 | 2-Chlor-5-methylpyridin | 0,1% (v/v) | 16 | 2-Chlor-5-hydroxymethylpyridin | 80 |
| 16 | 2,5-Dimethylpyrazin | 0,1% (v/v) | 16 | 2-Hydroxymethyl-5-methylpyrazin | 50 |
| 17 | 2,6-Dimethylpyrimidin | 0,1% (w/v) | 16 | 2-Hydroxymethyl-4-methylpyrimidin | 10 |
| 18 | 3,5-Dimethylpyrazol | 0,1% (w/v) | 16 | 3-Hydroxymethyl-6-methylpyrazol | 10 |
| 19 | 4-Methylthiazol | 0,1% (v/v) | 16 | 4-Hydroxymethylthiazol | 10 |
| 20 | 2,5-Dimethylthiophen | 0,1% (v/v) | 16 | 2-Hydroxymethyl-5-methylthiophen | 10 |

## Patentansprüche

1. Mikrobiologisches Verfahren zur Hydroxylierung von Methylgruppen in aromatischen Heterocyclen, dadurch gekennzeichnet, dass man die Umsetzung mit Mikroorganismen durchführt, die

a) die Gene von einem Pseudomonas TOL-Plasmid enthalten, die eine aktive Xylol-Monooxygenase bilden,

b) keine wirksame chromosomal- oder plasmidcodierte Alkoholdehydrogenase bilden

und damit befähigt sind, Methylgruppen von aromatischen 5- oder 6-Atom-Heterocyclen zum entsprechenden Hydroxymethylderivat zu hydroxylieren, wobei der Heterocyclus als Substrat für die Umsetzung dient und keinen Substituenten am benachbarten Kohlenstoffatom der zu hydroxylierenden Methylgruppe aufweist und das Hydroxymethylderivat nicht weiter katabolisiert wird.

2. Verfahren nach Patentanspruch 1, dadurch gekennzeichnet, dass man die Umsetzung mit Mikroorganismen durchführt bei denen die Gene von einem Pseudomonas TOL-Plasmid stammen, die eine aktive Xylol-Monooxygenase bilden und durch nachstehende Restriktionskarte charakterisiert sind:

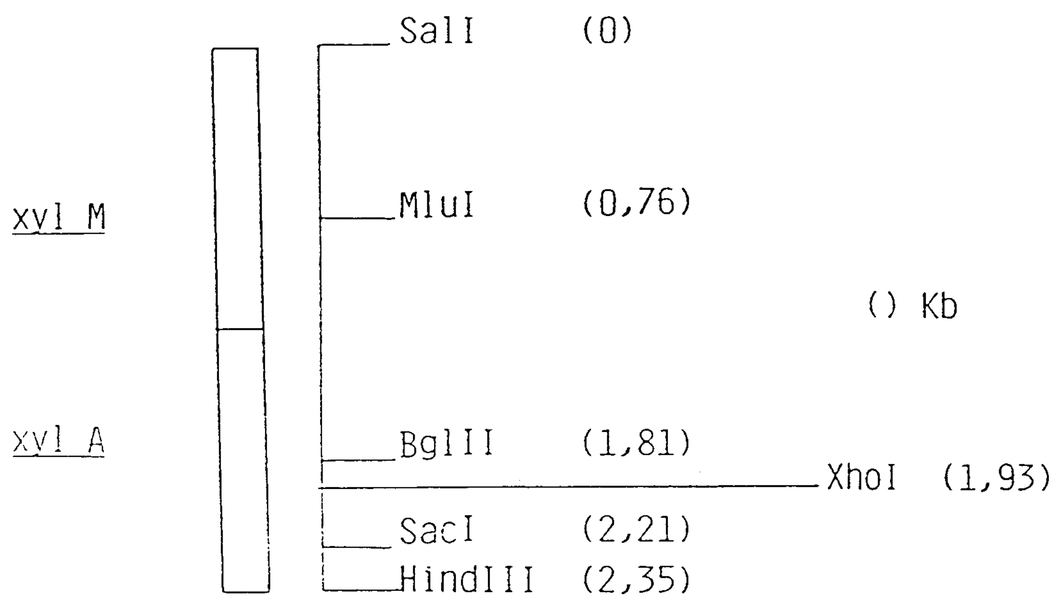


3. Verfahren nach nach mindestens einem der Patentansprüche 1 bis 2, dadurch gekennzeichnet, dass man die Umsetzung mit Mikroorganismen durchführt, die der Gattung Pseudomonas, Acinetobacter, Rhizobium, Agrobacterium oder Escherichia angehören.

4. Verfahren nach Patentanspruch 1, dadurch gekennzeichnet, dass man die Umsetzung mit Mikroorganismen durchführt, die ein natürliches TOL-Plasmid enthalten, in denen das Gen, welches für eine wirksame Alkohol-Dehydrogenase kodiert, entfernt oder inaktiviert ist.

5. Verfahren nach mindestens einem der Patentansprüche 1 bis 3, dadurch gekennzeichnet, dass man die Umsetzung mit dem Mikroorganismus der Spezies Escherichia coli K12*, transformiert mit dem Hybridplasmid pGSH2836 (DSM-Nr. 6154) oder mit dessen Deszendenten und Mutanten durchführt.

6. Verfahren nach mindestens einem der Patentansprüche 1 bis 3, dadurch gekennzeichnet, dass man die Umsetzung mit dem Mikroorganismus der Spezies Escherichia coli K12* transformiert mit dem Hybridplasmid pLO4 (DSM-Nr. 6153) oder mit dessen Deszendenten und Mutanten, durchführt.

7. Verfahren nach mindestens einem der Patentansprüche 1 bis 3, dadurch gekennzeichnet, dass man die Umsetzung mit dem Mikroorganismus der Spezies Pseudomonas putida JD7 transformiert mit dem Hybridplasmid pLO5 (DSM-Nr 6152) oder mit dessen Deszendenten und Mutanten, durchführt.

8. Verfahren nach mindestens einem der Patentansprüche 1 bis 7, dadurch gekennzeichnet, dass man die Umsetzung mit einem methylierten 5- oder 6-Atom-Heterocyclus durchführt, der ein oder mehrere Heteroatome aus der Reihe Sauerstoff, Stickstoff, Schwefel enthält.

9. Verfahren nach mindestens einem der Patentansprüche 1 bis 8, dadurch gekennzeichnet, dass man die Umsetzung entweder unter einmaliger oder kontinuierlicher Substratzugabe durchführt, so dass die Substratkonzentration im Kulturmedium 20% (w/v) oder (v/v) nicht übersteigt.

10. Verfahren nach mindestens einem der Patentansprüche 1 bis 9, dadurch gekennzeichnet, dass man die Umsetzung bei einem pH von 4 bis 11 und bei einer Temperatur von 15 bis 50 ° C durchführt.

11. Hybridplasmid pLO4 bestehend aus Expressionsvektor pMMB67EH und den Genen von einem Pseudomonas TOL-Plasmid gemäss Patentanspruch 2, mit einer Länge von 2,35 Kb, die eine SalI- und eine HindIII-Schnittstelle aufweisen, wie hinterlegt in Escherichia coli K12* (DSM-Nr. 6153).

12. Hybridplasmid pLO5 bestehend aus Expressionsvektor pMMB67EH* und den Genen von einem Pseudomonas TOL-Plasmid gemäss Patentanspruch 2, mit einer Länge von 2,35 Kb, die eine SalI- und eine HindIII-Schnittstelle aufweisen, wie hinterlegt in Pseudomonas putida JD7 (DSM-Nr. 6152).

13. Mikroorganismus der Spezies Escherichia coli K12* transformiert mit dem Hybridplasmid pLO4 (DSM-Nr. 6153) und dessen Deszendenten und Mutanten.

14. Mikroorganismus der Spezies Pseudomonas putida JD7 transformiert mit dem Hybridplasmid pLO5 (DSM-Nr. 6152) und dessen Deszendenten und Mutanten.

**Claims**

1. A microbiological process for hydroxylating methyl groups in aromatic heterocyclic compounds, characterized in that the conversion is carried out by microorganisms which
   a) contain the genes of a Pseudomonas TOL-plasmid which express an active xylene monooxygenase,
   b) do not form an active chromosomal or plasmid encoded alcohol dehydrogenase

   and thus are capable of hydroxylating methyl groups of aromatic 5- or 6-membered heterocyclic compounds to form the corresponding hydroxymethyl derivative, wherein the heterocyclic compound serves as a substrate for the conversion and has no substituent on the carbon atom next to the methyl group to be hydroxylated and wherein the hydroxymethyl derivative is not further catabolized.

2. Process according to claim 1, characterized in that the conversion is carried out by microorganisms whose genes forming an active xylene monooxygenase are derived from a Pseudomonas TOL-plasmid and are characterized by the following restriction map:

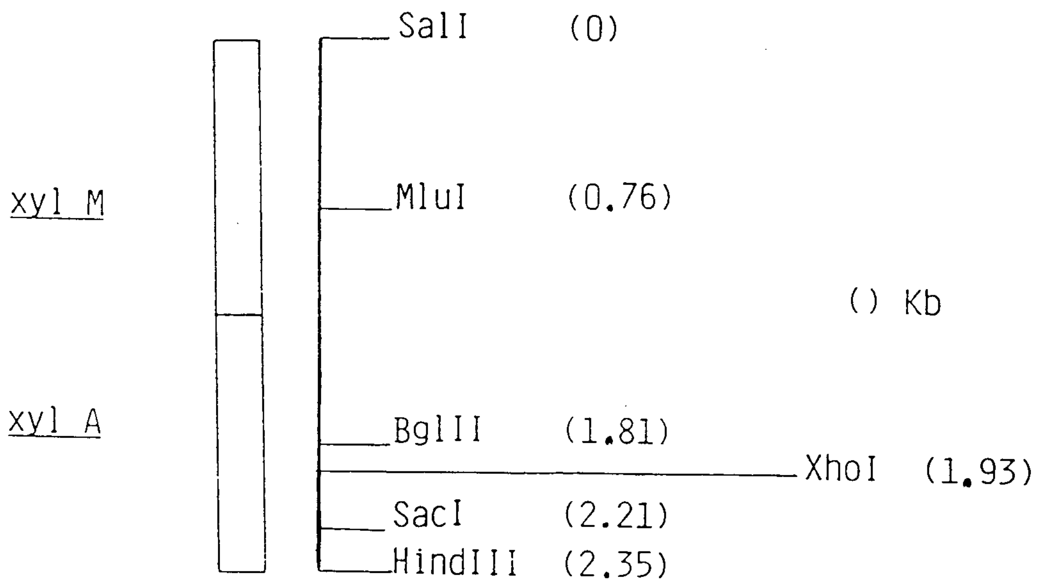

3. Process according to at least one of claims 1 to 2, characterized in that the conversion is carried out by microorganisms of the genus Pseudomonas, Acinetobacter, Rhizobium, Agrobacterium, or Escherichia.

4. Process according to claim 1, characterized in that the conversion is carried out by microorganisms containing a native TOL-plasmid in which the gene coding for an active alcohol dehydrogenase is deleted or inactivated.

5. Process according to at least one of claims 1 to 3, characterized in that the conversion is carried out by a microorganisms of the species Escherichia coli K12* transformed by hybrid plasmid pGSH2836 (DSM-No. 6154) or descendants or mutants thereof.

6. Process according to at least one of claims 1 to 3, characterized in that the conversion is carried out by a microorganisms of the species Escherichia coli K12* transformed by hybrid plasmid pLO4 (DSM-No. 6153) or descendants or mutants thereof.

7. Process according to at least one of claims 1 to 3, characterized in that the conversion is carried out by a microorganisms of the species Pseudomonas putida JD7 transformed by hybrid plasmid pLO5 (DSM-No. 6152) or descendants or mutants thereof.

8. Process according to at least one of claims 1 to 7, characterized in that the conversion is carried out with a methylated 5- or 6-membered heterocylic compound containing one or more heteroatoms of the group of oxygen, nitrogen and sulphur.

9. Process according to at least one of claims 1 to 8, characterized in that the conversion is carried out by adding the substrate either once or continuously so that the substrate concentration in the culture medium does not exceed 20% (w/v) or (v/v).

10. Process according to at least one of claims 1 to 9, characterized in that the conversion is carried out at a pH of from 4 to 11 and at a temperature of from 15 to 50 °C.

11. Hybrid plasmid pLO4, consisting of expression vector pMMB67EH and of the genes of a Pseudomonas TOL-plasmid according to claim 2 having a size of 2.35 kb and a SalI and a HindIII restriction site as deposited in Escherichia coli K12* (DSM-No. 6153).

12. Hybrid plasmid pLO5, consisting of expression vector pMM67EH* and of the genes of a Pseudomonas TOL-plasmid according to claim 2 having a size of 2.35 kb and a SalI and a HindIII restriction site as deposited in Pseudomonas putida JD7 (DSM-No. 6152).

13. Microorganism of the species Escherichia coli K12* transformed by hybrid plasmid pLO4 (DSM-No. 6153) and descendants and mutants thereof.

14. Microorganism of the species Pseudomonas putida JD7 transformed by hybrid plasmid pLO5 (DSM-No. 6152) and descendants and mutants thereof.

## Revendications

1. Procédé microbiologique d'hydroxylation de groupes méthyle dans des hétérocycles aromatiques, caractérisé en ce que l'on effectue la réaction avec des microorganismes qui

   a) contiennent les gènes d'un plasmide TOL de *Pseudomonas*, qui forment une xylène monooxygénase active,

   b) ne forment pas d'alcool déshydrogénase active codée par un gène chromosomique ou plasmidique,

   et sont ainsi capables d'hydroxyler des groupes méthyle d'hétérocycles aromatiques de 5 ou 6 atomes en les dérivés hydroxyméthylés correspondants, l'hétérocycle servant de substrat pour la réaction et ne présentant pas de substituant sur l'atome de carbone voisin du groupe méthyle à hydroxyler, et le dérivé hydroxyméthylé ne subissant pas d'autre catabolisme.

2. Procédé selon la revendication 1, caractérisé en ce que l'on effectue la réaction avec des microorganismes dans lesquels les gènes qui forment une xylène monooxygénase active proviennent d'un plasmide TOL de *Pseudomonas* et sont caractérisés par la carte de restriction suivante:

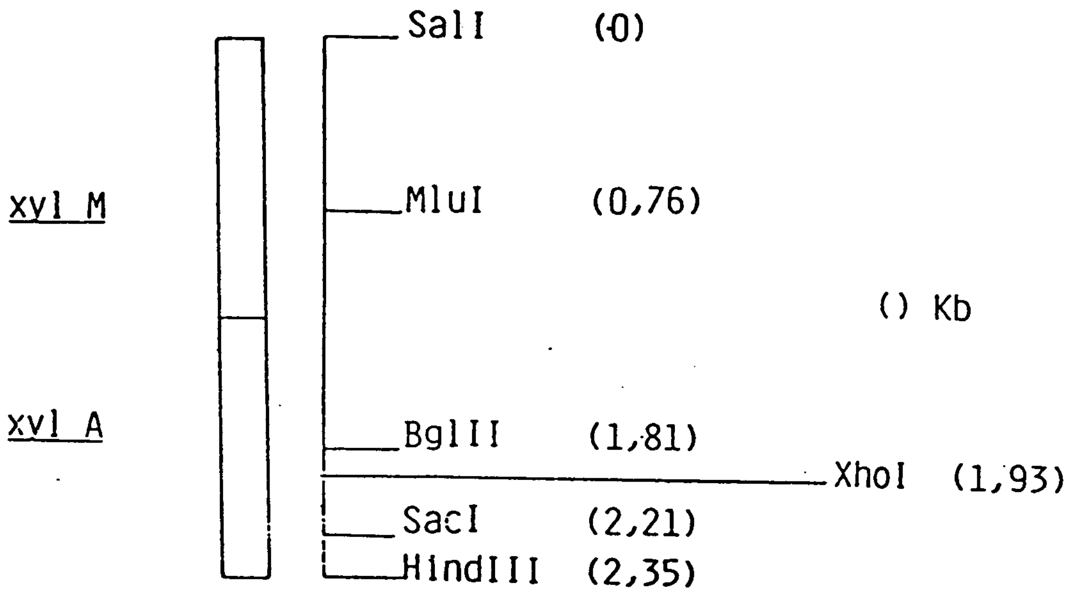


3. Procédé selon au moins l'une des revendications 1 et 2, caractérisé en ce que l'on effectue la réaction avec des microorganismes qui appartiennent au genre *Pseudomonas, Acinetobacter, Rhizobium, Agrobacterium* ou *Escherichia*.

4. Procédé selon la revendication 1, caractérisé en ce que l'on effectue la réaction avec des microorganismes qui contiennent un plasmide TOL naturel dans lequel le gène codant pour une alcool déshydrogénase active est supprimé ou inactivé.

5. Procédé selon au moins l'une des revendications 1 à 3, caractérisé en ce que l'on effectue la réaction avec le microorganisme de l'espèce *Escherichia coli* K12*, transformé avec le plasmide hybride pGSH2836 (DSM n° 6154) ou avec ses descendants et mutants.

6. Procédé selon au moins l'une des revendications 1 à 3, caractérisé en ce que l'on effectue la réaction avec le microorganisme de l'espèce *Escherichia coli* K12*, transformé avec le plasmide hybride pLO4 (DSM n° 6153) ou avec ses descendants et mutants.

7. Procédé selon au moins l'une des revendications 1 à 3, caractérisé en ce que l'on effectue la réaction avec le microorganisme de l'espèce *Pseudomonas putida* JD7 transformé avec le plasmide hybride pLO5 (DSM n° 6152) ou avec ses descendants et mutants.

8. Procédé selon au moins l'une des revendications 1 à 7, caractérisé en ce que l'on effectue la réaction avec un hétérocycle de 5 ou 6 atomes méthylé contenant un ou plusieurs hétéroatomes de la série de l'oxygène, de l'azote et du soufre.

9. Procédé selon au moins l'une des revendications 1 à 8, caractérisé en ce que l'on effectue la réaction en ajoutant le substrat en une fois ou en continu de façon la concentration de substrat dans le milieu de culture ne dépasse pas 20 % (m/v) ou (v/v).

10. Procédé selon au moins l'une des revendications 1 à 9, caractérisé en ce que l'on effectue la réaction à un pH de 4 à 11 et à une température de 15 à 50 °C.

11. Plasmide hybride pLO4 constitué par le vecteur d'expression pMMB67EH et les gènes d'un plasmide TOL de *Pseudomonas* selon la revendication 2, ayant une longueur de 2,35 kb, contenant un site de

coupure par *Sal*I et un site de coupure par *Hin*dIII, tel que déposé dans *Escherichia coli* K12* (DSM n° 6153).

12. Plasmide hybride pLO5 constitué par le vecteur d'expression pMMB67EH* et les gènes d'un plasmide TOL de *Pseudomonas* selon la revendication 2, ayant une longueur de 2,35 kb, contenant un site de coupure par *Sal*I et un site de coupure par *Hin*dIII, tel que déposé dans *Pseudomonas putida* JD7 (DSM n° 6152).

13. Microorganisme de l'espèce *Escherichia coli* K12* transformé avec le plasmide hybride pLO4 (DSM n° 6153) et ses descendants et mutants.

14. Microorganisme de l'espèce *Pseudomonas putida* JD7 transformé avec le plasmide hybride pLO5 (DSM n° 6152) et ses descendants et mutants.